(19) Europäisches Patentamt / European Patent Office / Office européen des brevets

(11) **EP 4 723 033 A1**

(12) **EUROPEAN PATENT APPLICATION**

(43) Date of publication:
08.04.2026 Bulletin 2026/15

(21) Application number: 24465581.7

(22) Date of filing: 02.10.2024

(51) International Patent Classification (IPC):
*G06T 7/00* (2017.01)    *A61B 6/50* (2024.01)
*G06V 10/82* (2022.01)

(52) Cooperative Patent Classification (CPC):
**G06T 7/0012; A61B 6/505; A61B 6/5217;**
**G06V 10/82;** A61B 6/032; A61B 6/4085;
A61B 6/463; G06T 2207/10081; G06T 2207/10088;
G06T 2207/10116; G06T 2207/20081;
G06T 2207/20084; G06T 2207/30012

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: Siemens Healthineers AG
91301 Forchheim (DE)

(72) Inventors:
• **Chekkoury, Andrei**
**91054 Erlangen (DE)**
• **Eibenberger, Eva**
**90427 Nürnberg (DE)**
• **Gibson, Eli**
**Lawrenceville, 08648 (US)**
• **Sandu, Andreea Elena**
**500371 Brasov (RO)**
• **Soza, Grzegorz**
**90562 Heroldsberg (DE)**

(74) Representative: **Siemens Healthineers**
**Patent Attorneys**
**Postfach 22 16 34**
**80506 München (DE)**

(54) **A COMPUTER IMPLEMENTED METHOD FOR DETERMINING A RESPECTIVE STATE OF PREDEFINED ANATOMICAL OBJECTS OF AN ANATOMICAL REGION, A COMPUTER IMPLEMENTED METHOD FOR TRAINING A FUNCTION, A DATA PROCESSING DEVICE, A MEDICAL IMAGING SYSTEM, A COMPUTER PROGRAM PRODUCT AND A COMPUTER READABLE MEDIUM**

(57) The invention relates to a computer-implemented method for determining a respective state (32) of predefined anatomical objects (14) of an anatomical region (16), comprising: receiving an image data set (22) of the anatomical region (16) comprising the predefined anatomical objects (14) by an input interface of a data processing device (12), identifying by a computation unit of the data processing device (12) respective image regions (30) of the image data set (22) depicting the predefined anatomical objects (14); applying a function trained by a machine learning algorithm to the respective image regions (30) depicting the respective predefined anatomical objects (14), wherein each of the image regions (30) is examined for a presence of a predetermined pathological finding in the respective anatomical objects (14).

FIG 1

EP 4 723 033 A1

**Description**

The present invention relates to a computer implemented method for determining a respective state of a plurality of predefined anatomical objects of an anatomical region, a computer implemented method for training a function, a data processing device for performing the method for determining a respective state of a plurality of predefined anatomical objects of an anatomical region a medical imaging system comprising the data processing device, a computer program product as well as a computer readable medium.

The present invention is in the field of medical imaging analysis, which is used for of creating data based representations, in particular visual representations, of regions of a body of a patient based on the detection of physical signals to obtain imaging data and imaging data-based analysis of the resultant imaging data to support analysis, in particular visual representation, of the function of organs or tissues.

Cervical spine (c-spine) fractures refer to injuries affecting the seven vertebras belonging to the neck region of the spine. The fractures range from minute and only cover small anatomical structures of single vertebras to severe fractures that involve entire vertebral body and even complete dislocations. Imaging of cervical spine is an essential component of diagnosing cervical spine fractures.

The imaging modality of choice for patients involved in high-velocity accidents, falls, shallow water dives, etc. is CT, as it has excellent sensitivity in visualizing fractures. The cervical spine of these poly-traumatic patients is stabilized by the first responders using an immobilization collar (i.e., a so-called stiffneck), immobilizing the cervical spine to prevent further injury to the cervical spine and neurological damage. Timely and accurately identifying cervical fractures in the polytraumatic patient is of utmost importance.

Computer aided triage (CADt) systems flagging cases positive for cervical spine fractures exist already, but have two main limitations:
Once a case is triaged, a positive image is generated without explicitly indicating the location of the finding. A generic, case level statement is made (e.g.," Suspected cervical spine fracture found")

These algorithms operate in a parallel environment, disjoint of the CT Scan Console where the user is doing the initial critical diagnosis. These kinds of implementations can potentially delay reporting of critical findings.

Currently, the radiologist must always clear the scan of any possible fractures before the stiff neck can be removed and the patient can be moved to the operating theatre. This is done based on freshly reconstructed images on the CT console under a lot of time and peer pressure.

Current CADt products generate a case-level label for positive findings (Aidoc). The user is generally confronted with a "needle in a haystack" type of problem trying to identify the location of the fracture. Furthermore, false-positive cases create confusion and possibly introduce additional delay in reporting of more urgent cases as the user is still looking for that "fracture found by AI".

There are two possible solutions to the above-listed limitations:
Implemented by Aidoc in a competitor product: Once a case is triaged, a not for diagnostic use image is generated showing a heat map image. The user must mentally register the CT image to the heat map image to interpret this heat-map image and identify the location of a possible fracture. This requires training, understanding of the AI results and implies using images intended for non-diagnostic purposes for explaining a positive CADt finding in the clinical context.

Indicating the location of a vertebral fracture can be done by developing a computer-aided detection and localization (CADe) medical product (not yet done for this pathology). This requires exhaustive efforts on performing clinical studies meant to collect evidence showcasing the superiority and the benefits of such a medical device. This approach is coupled with a considerable financial burden and negatively impacts the time to market of such a solution.

US 2022/0309667 A1 describes an automatic hemorrhage expansion detection from head ct images.

US 11 275 976 B2 describes a medical image assessment with classification uncertainty.

US 2022/0293247 A1 describes a machine learning for automatic detection of intracranial hemorrhages with uncertainty measures from ct images.

It is an objective of the present invention to provide a method for determining a respective state of predefined a plurality of anatomical objects of an anatomical region to support a physician analyzing the image data set.

This objective is achieved by the subject-matter of the independent claims. Further implementations and preferred embodiments are subject-matter of the dependent claims.

A first aspect of the present invention relates to a computer-implemented method for determining a respective state of predefined a plurality of anatomical objects of an anatomical region. The method begins with receiving an image data set of the anatomical region comprising the predefined anatomical objects by an input interface of a data processing device. This image data set is accessible from a data storage device or is provided by a medical imaging system such as a CT-Device or an MRI device. The image data set is then processed to identify respective image regions of the image data set depicting the predefined anatomical objects using the computation unit of the data processing device.

In a next step, a function trained by a machine learning algorithm is applied to the respective image regions depicting the respective predefined anatomical objects. Each of the image regions is examined for a presence of a predetermined pathological finding in the respective anatomical objects. The application of this machine learning algorithm allows for the identification and analysis of complex patterns within the image data set that may indicate the presence of a pathological finding.

After analyzing each image region, the method determines for each predefined anatomical object of the plurality of predefined anatomical objects a respective state based on the presence of the predetermined pathological finding in the respective predefined anatomical object. This determination may be used to support a physician in their analysis of the image data set to identify any predetermined pathological findings in the anatomical objects.

Finally, the respective states of the predefined anatomical objects are provided by a second interface of the data processing device. This allows for easy interpretation and understanding of the results of the analysis, enabling the physician to make informed decisions regarding any necessary medical interventions.

In summary, the present invention provides a computer-implemented method for determining the respective states of predefined anatomical objects within an image data set. The use of machine learning algorithms allows for the identification and analysis of complex patterns within the image data set, providing valuable support to physician's in their analysis of medical imaging data. In contrast to known prior art, the method described enables the physician to be alerted not only to the presence of a pathological findings, but also to the relevant anatomical objects. The physician can thus concentrate their time on the examination of the relevant anatomical object.

Unless stated otherwise, all steps of the computer-implemented method according to the first aspect of the present invention may be performed by the data processing device. In particular, the data processing device is configured or adapted to perform the steps of the computer-implemented method. For this purpose, the data processing device may for example store a computer program comprising instructions which, when executed by the data processing device, cause the data processing device to execute the computer-implemented method. In other words, the data processing device according to the invention can be improved with features described or claimed in the context of the methods of the invention and vice versa. In this case, the functional features of the method are embodied by objective units or modules of the data processing device.

According to a further embodiment of the present invention, the computer-implemented method for providing a trained function comprises generating visual representation data for a display device by the computation unit of the data processing device. This visual representation data comprises a visualization of at least part of the anatomical region, which includes at least one of the image regions depicting the respective anatomical object. The visualization depends on the state of at least one of the predefined anatomical objects. The visual representation data is then provided through the second interface of the data processing device. The visual representation data may be displayed on a display device for further analysis by the medic. The visual representation data may show the anatomical region, highlighting image regions depicting the respective anatomical object comprising relevant pathological findings.

According to a further embodiment of the present invention, the respective states of the predefined anatomical objects describe a confidence value and/or a probability value of the presence of the predetermined pathological finding in the respective anatomical object. The determination of the confidence value and/or probability value allows for a more informed analysis of the medical image by the medic. The state of each predefined anatomical object is described by these values, which reflect the likelihood of the presence of a predetermined pathological finding in that particular anatomical object. The probability value may provide additional information about the likelihood of the presence of the pathological finding in the respective anatomical object. The confidence value is described in US 11 275 976 B2. The confidence value, in the context of classifying the image data set using deep learning models, may refer to a measure of uncertainty that indicates a level of certainty or conviction of the model in its predicted classification. It is an orthogonal measurement, meaning it is separate from the probabilistic estimate of the presence or absence of the pathological finding. The confidence value may be learned during training, where a deep learning network is trained not only to predict the probability of the pathological finding but also to quantify the uncertainty in its classification. This allows the model to account for the inherent variability and ambiguity of image data sets, providing a more nuanced and reliable prediction. This design has the advantage of recognizing that the presence of the pathological finding cannot always be clearly established.

According to a further embodiment of the present invention, the computer-implemented method is specifically designed for analyzing the imaging data set representing a cervical spine. In this embodiment, the anatomical region of interest is the cervical spine, and the predefined anatomical objects are the vertebral bodies of the cervical spine. The cervical spine is a structure comprising seven of the vertebral bodies. The vertebral bodies support a head and enable movement of a neck. Pathological findings in the cervical spine may have serious consequences for patient health, making accurate analysis of the imaging data set essential for proper diagnosis and treatment planning.

According to a further embodiment of the present invention, the predetermined pathological finding is specifically a fracture of the respective vertebral body of the cervical spine. Vertebral fractures are a common and serious type of spinal injury that may have significant consequences for patient health, making accurate diagnosis essential for proper treatment planning. In other words, according to the embodiment of the present invention, the computer-implemented method is specifically designed for analyzing the imaging data set to determine the presence or an absence of the vertebral fractures in the cervical spine. The embodiment assists the physician in finding present pathological findings in the cervical spine.

The embodiment is advantageous because, in the case of present cervical spine fractures, rapid diagnosis is essential for proper treatment, reducing the risk of further injury or long-term complications.

According to a further embodiment of the present invention, the image data set is a set of CT images. In other words, the computer-implemented method is specifically designed for use with CT (computed tomography) images as the image data set. CT imaging is a commonly used medical imaging technique that uses X-ray technology and computer processing to generate detailed cross-sectional images of the body, making it an ideal tool for diagnosing a wide range of pathological conditions in the spine. The CT images are provided by a medical imaging system such as a CT-device.

According to a further embodiment of the present invention, the image data set is a set of MRI images. In other words, the computer-implemented method is specifically designed for use with MRI (magnetic resonance imaging) images as the image data set. MRI is a non-invasive medical imaging technique that uses strong magnetic fields and radio waves to create detailed cross-sectional images of the body, making it an ideal tool for diagnosing a wide range of pathological conditions in the spine.

According to a further embodiment of the present invention, the image data set comprises one or more X-ray images. In other words, the computer-implemented method is specifically designed for use with at least one X-ray image as the image data set. X-ray imaging is a commonly used medical imaging technique that uses low doses of radiation to create detailed images of the body's internal structures, making it an ideal tool for diagnosing skeletal conditions and fractures in the spine.

According to a further embodiment of the present invention, the computer-implemented method comprises acquiring the image data set using a medical imaging device. In other words, the method comprises the generation of the image data set that is analyzed in the method. The medical imaging device may be any device that is configured to generate the image data set of the anatomical region, such as a CT-Device or an MRI device. The data processing device may be part of the medical imaging device and/or external to the medical imaging device.

The acquisition of the image data set by the medical imaging device may involve several steps, comprising image capture, pre-processing, and quality control. During image capture, the patient may be positioned on the imaging device, and images may be taken at specific angles and orientations to ensure complete coverage of the anatomical region. Pre-processing may involve correcting for any artifacts or noise in the images, while quality control ensures that the images meet the required standards for analysis.

Once the images have been acquired, the image data set is provided to the data processing device by the medical imaging device. The image data set may be provided via a direct data connection like an Ethernet connection. Alternatively, the image data set may be stored on a storage device and accessed by the data processing device from the storage device.

A second aspect of the present invention relates to a computer-implemented training method for providing a trained function. The method comprises receiving input training data by a first training interface of a training device, said input training data comprising image data sets of respective anatomical regions comprising predefined anatomical objects, wherein each image data set comprises image regions depicting the predefined anatomical objects. The method comprises receiving output training data by a second training interface of the training device, said output training data comprising for each image region an indication of a presence of a predetermined pathological finding in the respective anatomical objects. In other words, the output training data comprises indications for each image region regarding the presence of the predetermined pathological finding in the respective anatomical objects.

In other words, a trained function can be trained with imaging data as input training data, wherein the input training data is associated or annotated with output training data with additional training information. The additional training information can comprise a ground truth information. As a non-limiting list of examples, the ground truth information can comprise a classification information which classifies a target region of the imaging data or a respective anatomical object in the imaging data into one of a plurality of classes, a scoring information, which associates a target region or a respective anatomical object in the imaging data with a score (e.g. a disease severity score or a probability score), an image segmentation information, a finding information indicative of the presence or absence of a clinical finding in a target region or a respective anatomical object in the imaging data, or a finding information of the presence or absence of a finding in an anatomical object in the imaging data. The association or annotation of the output training data can be performed manually by trained personnel and/or automatically by use or with the aid of a respective image analysis software.

The method further comprises training a function based on the input training data and the output training data by a training computation unit of the training device, and providing the trained function by a third training interface of the training device. The image data sets may be provided by a medical imaging system like a CT-Device or an MRI device.

The function is trained based on patterns within the image data sets of the input training data and their corresponding indications of predetermined pathological findings, given in the output training data, and is provided through a third interface. The function is trained for use in diagnosing new image data sets. The function is trained for use in the method according to the first aspect of the present invention.

In general, a trained function mimics cognitive functions that humans associate with other human minds. In particular, by training based on training data the trained function is able to adapt to new circumstances and to detect and extrapolate patterns.

In general, parameters of a trained function can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained functions can be adapted iteratively by several steps of training.

In particular, a trained function can comprise a neural network, a support vector machine, a decision tree and/or a Bayesian network, and/or the trained function can be based on k-means clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

According to a third aspect of the invention, a data processing device is provided, which is configured to perform a computer-implemented method according to the first aspect of the present invention.

The data processing device may in particular be understood as a data processing device, which comprises processing circuitry. The data processing device can therefore in particular process data to perform computing operations. This may also include operations to perform indexed accesses to a data structure, for example a look-up table, LUT, as well as a data processing process implemented in hardware.

In particular, the data processing device may include one or more computers, one or more microcontrollers, and/or one or more integrated circuits, for example, one or more application-specific integrated circuits, ASIC, one or more field-programmable gate arrays, FPGA, and/or one or more systems on a chip, SoC. The data processing device may also include one or more processors, for example one or more microprocessors, one or more central processing units, CPU, one or more graphics processing units, GPU, and/or one or more signal processors, in particular one or more digital signal processors, DSP. The data processing device may also include a physical or a virtual cluster of computers or other of said units.

In various embodiments, the data processing device includes one or more hardware and/or software interfaces and/or one or more memory units.

A memory unit may be implemented as a volatile data memory, for example a dynamic random access memory, DRAM, or a static random access memory, SRAM, or as a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to a fourth aspect of the invention, a medical imaging system is provided. The medical imaging system comprises a data processing device according to the third aspect of the present invention and a medical imaging device. The medical imaging device is configured to generate imaging raw data representing the anatomical region and to generate the image data set depending on the imaging raw data.

The medical imaging device may, for example, be a CT device, a CBCT device, an MRI device or an X-ray device.

According to a fifth aspect of the present invention, a computer program product comprising instructions is provided. When the instructions are executed by a data processing device, the instructions cause the data processing device to carry out a computer-implemented method according to the first and/or the second aspect of the present invention. The instructions may be provided as program code, for example. The program code can for example be provided as binary code or assembler and/or as source code of a programming language, for example C, and/or as program script, for example Python.

According to a sixth aspect of the invention, a computer-readable storage medium, in particular a tangible and/or non-transient computer readable storage medium, storing a computer program according to the invention is provided. The computer program and the computer-readable storage medium are respective computer program products comprising instructions. The instructions cause the data processing device to carry out a computer-implemented method according to the first and/or the second aspect of the present invention.

Independent of the grammatical term usage, individuals with male, female or other gender identities are included within the term patient.

Further features and feature combinations of the invention are obtained from the figures and their description as well as the claims. In particular, further implementations of the invention may not necessarily contain all features of one of the claims. Further implementations of the invention may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the Figures,

FIG 1 shows a schematic illustration of a medical imaging system;

FIG 2 shows a schematic illustration of a visualization of at least part of the anatomical region of the visual representation data;

FIG 3 shows a schematic flow diagram of a computer-implemented method for determining a respective state of predefined anatomical objects of an anatomical region;

FIG 4 shows a schematic flow diagram of a computer-implemented method for providing a trained function;

FIG 5 a schematic illustration of an artificial neural network; and

FIG 6 a schematic illustration of convolutional neural network.

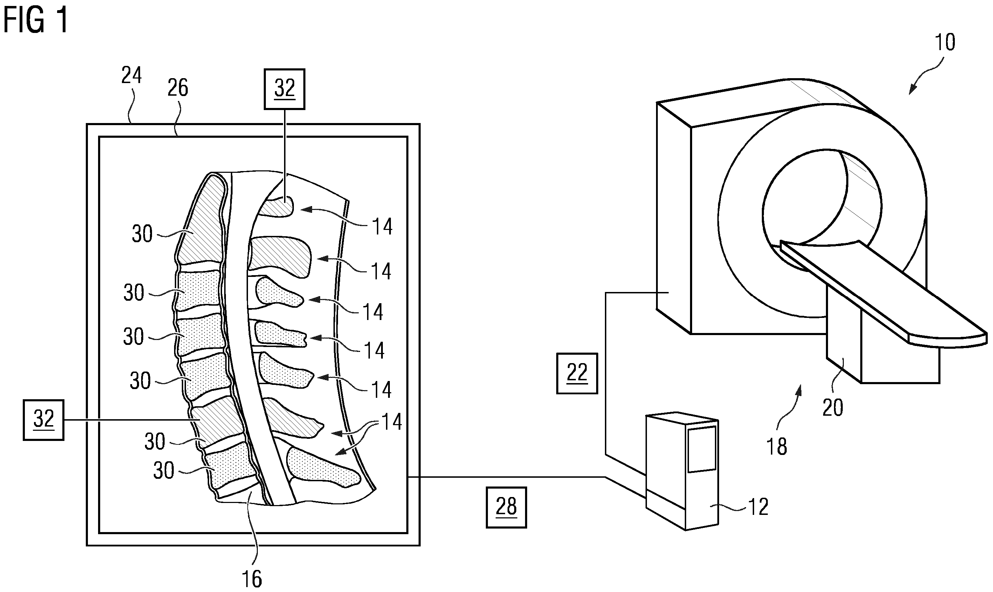

FIG 1 shows a schematic illustration of a medical imaging system 10.

The medical imaging system 10 may comprise a data processing device 12, which may be configured to carry out a computer-implemented method for determining a respective state 32 of predefined anatomical objects 14 of an anatomical region 16. The medical imaging system 10 may also comprise a medical imaging device 18, which may for example be a CT-device or an MR-device. Optionally, the medical imaging system 10 comprises a patient table 20 or the like. The medical imaging device 18 is configured to generate an image data set 22 representing the anatomical region 16, of a body on the patient table 20. The image data set 22 may comprise a three-dimensional or a two-dimensional representation of the anatomical region 16. The anatomical region 16 may be a cervical spine of the body.

The image data set 22 of the anatomical region 16 may be generated to support a physician to examine the image data set 22 for a presence of a predetermined pathological finding in the anatomical objects 14 of the anatomical region 16. The anatomical objects 14 may be vertebral bodies of the cervical spine. The medical imaging system 10 may comprise a display device 24 to show a visual representation 26 of the image data set 22 described in visual representation data 28 generated by the data processing device 12.

To support the analysis of the anatomical objects 14 in the anatomical region 16, the image data set 22 may be provided to the data processing device 12 to perform a computer-implemented method for determining the respective states 32 of the predefined anatomical objects 14 of the anatomical region 16 on the image data set 22.

The data processing device 12 may be configured to receive the image data set 22 of the anatomical region 16 comprising the predefined anatomical objects 14 at an input interface of the data processing device 12. The input interface may be connected to a respective output interface of the medical imaging device 18. The data processing device 12 may be configured to identify by a computation unit of the data processing device 12 respective image regions 30 of the image data set 22 depicting the predefined anatomical objects 14. The respective image regions 30 may be identified by using image analysis procedures according to the state 32 of the art. In the specific case, the data processing device 12 may be configured to identify the image regions 30 showing the predefined anatomical objects 14. The predefined anatomical objects 14 may be seven defined vertebral bodies of the cervical spine. The image regions 30 may be three-dimensional regions or two-dimensional regions.

The data processing device 12 may be configured to examine each of the image regions 30 for a presence of a predetermined pathological finding in the respective anatomical objects 14. The predetermined pathological finding may comprise fractures of the respective vertebral bodies. The data processing device 12 may be configured to examine the respective predefined anatomical objects 14 by applying a function trained by a machine learning algorithm to the respective image regions 30 depicting the respective predefined anatomical objects 14. The examination may be performed on each of the predefined anatomical objects 14 individually to determine whether the predetermined pathological finding is present in the respective of the anatomical objects 14.

The data processing device 12 may be configured to determine for each of the predefined anatomical objects 14 a respective state 32 based on the presence of the predetermined pathological finding in the respective predefined anatomical object 14. The respective state 32 of the anatomical object 14 may indicate a presence of the pathological finding in the respective predefined anatomical object 14, a number of the pathological findings in the respective predefined anatomical object 14 and/or a probability value giving a probability of the presence of the pathological finding in the respective predefined anatomical object 14 and/or a confidence value indicating a confidence of the presence of the pathological finding in the respective predefined anatomical object 14.

The data processing device 12 may be configured to provide the respective states 32 of the predefined anatomical objects 14 by a second interface of the data processing device 12. The second interface of the data processing device 12 may be connected to the medical imaging device 18.

The data processing device 12 may be configured to provide the visual representation 26 of the anatomical region 16 based on the image data set 22 of the anatomical region 16. The visual representation 26 may show the anatomical region 16 comprising the predefined anatomical objects 14, wherein the predefined anatomical objects 14 may be visualized. A visualization of the respective anatomical objects 14 may depend on their state 32. A visualization of the respective anatomical objects 14 may further depend on the pathological finding in the respective predefined anatomical object 14. The visual representation data 28 may be provided to the display device 24 and/or stored to a storage device.

Therefore it may be possible to overlay the anatomical region 16 comprising the respective predefined anatomical objects 14 with the results of the method. A physician may therefore focus on analyzing the respective predefined anatomical object 14 of specific states 32.

The problem of not having a clear indication of which vertebral level is affected by a fracture in a positively triaged cervical scan can be solved by performing a vertebral-level triage. The proposed method may treat the cervical spine triage problem as seven individual triaging problems and may analyze each of the individual vertebral body uniquely.

The results of the vertebral level triaging system may be displayed on the visual representation 26 showing an anatomical schematic. This method has two advantages: on one hand, the user is clearly aware of which of the vertebral bodies is detected as fractured by the trained algorithm. On the other hand, by performing a triage at a vertebral level, the visual representation 26 may be used for clinical purposes, as the triaging is still being done for a pathological finding, but on a more detailed level, on a vertebral level, and not on a generic cervical spine level. By using the visual representation 26 rather than a model derived from the image data set 22, the system is explicitly avoiding directing the user to a specific region of a specific image, which is outside of the scope of the CADt product category.

A problem of a disjoint processing unit can be solved by deploying the proposed solution directly on a console of the imaging system. This ensures much faster processing times, no DICOM transfer, and reduces the burden of integrating a new processing unit in a hospital's networking system. Moreover, anonymization concerns of certain clinics may be implicitly addressed, as data is not leaving the imaging system to be processed. Consequently, the result is available directly at the location of the patient, i.e., at the scanner and can be considered for patient handling and further diagnostic imaging, e.g., additional scan to diagnose potential vascular injuries due to fractures.

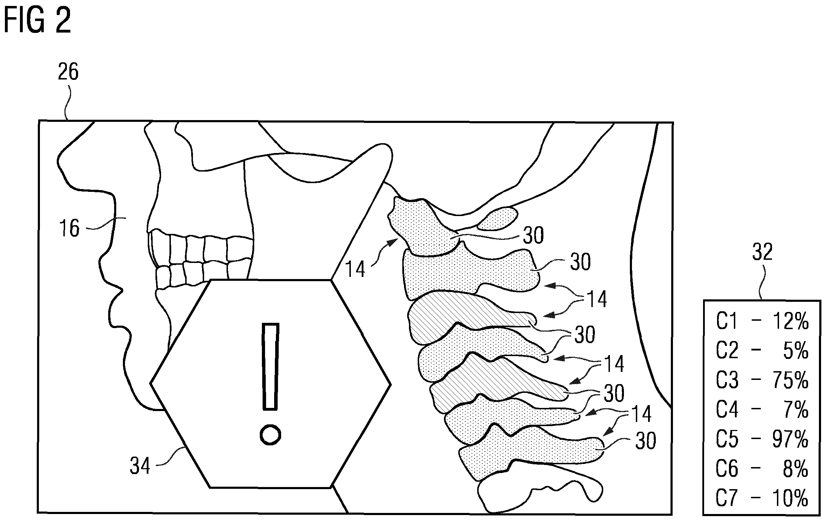

FIG 2 shows a schematic illustration of a visualization of at least part of the anatomical region 16 of the visual representation data 28.

The visual representation 26 may show the anatomical region 16 comprising the respective predefined anatomical objects 14. The respective predefined anatomical objects 14 may be highlighted in the representation. A surrounding, a color or another property of the respective anatomical objects 14 in the visual representation 26 may depend on their state 32. For each of the anatomical objects 14 a probability value or a confidence value may be shown in the visual representation 26. The visual representation 26 may comprise a sign 34 indicating a detection of a predetermined pathological finding in at least one of the anatomical objects 14.

On an alternative embodiment, the solution may be extended by the addition of confidence values. This solution addresses the transparency and the understandability of the solutions of the trained algorithm and provides a clearer visualization of the results.

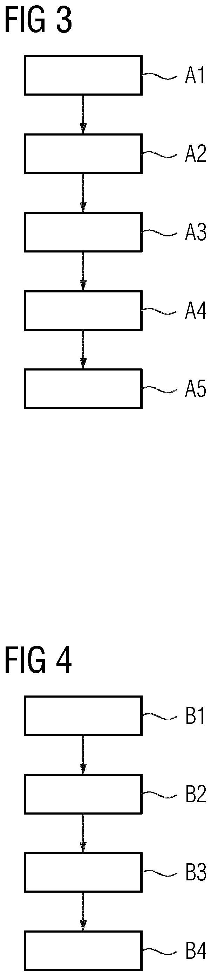

FIG 3 shows a schematic flow diagram of a computer-implemented method for determining a respective state 32 of predefined anatomical objects 14 of an anatomical region 16.

The computer-implemented method may be performed by the data processing device 12 shown in FIG 1.

The computer-implemented method for determining a respective state 32 of predefined anatomical objects 14 of an anatomical region 16 may comprise the following steps A1-A5.

A first step A1 may comprise receiving an image data set 22 of the anatomical region 16 comprising the predefined anatomical objects 14 by an input interface of a data processing device 12.

A second step A2 may comprise identifying by a computation unit of the data processing device 12 respective image regions 30 of the image data set 22 depicting the predefined anatomical objects 14.

A third step A3 may comprise applying a function trained by a machine learning algorithm to the respective image regions 30 depicting the respective predefined anatomical objects 14. Each of the image regions 30 may be examined for a presence of a predetermined pathological finding in the respective anatomical objects 14.

A fourth step A4 may comprise determining for each of the predefined anatomical objects 14 a respective state 32 based on the presence of the predetermined pathological finding in the respective predefined anatomical object 14.

A fifth step A5 may comprise providing the respective states 32 of the predefined anatomical objects 14 by a second interface of the data processing device 12.

FIG 4 shows a schematic flow diagram of a computer-implemented method for providing a trained function.

The computer-implemented method may be performed by a training device.

The computer-implemented method may comprise the following steps B1-B4.

A first step B1 may comprise receiving input training data comprising image data sets 22 of respective anatomical regions 16 comprising respective predefined anatomical objects 14 by a first training interface of the training device. The image data sets 22 comprise image regions 30 depicting the predefined anatomical objects 14.

A second step B2 may comprise receiving output training data by a second training interface of the training device. The output training data comprise for each of the image regions 30 an indication of a presence of a predetermined pathological finding in the respective anatomical objects 14.

A third step B3 may comprise training a function based on the input training data and the output training data by a training computation unit of the training device.

A fourth step B4 may comprise providing the trained function by a third training interface of the training device.

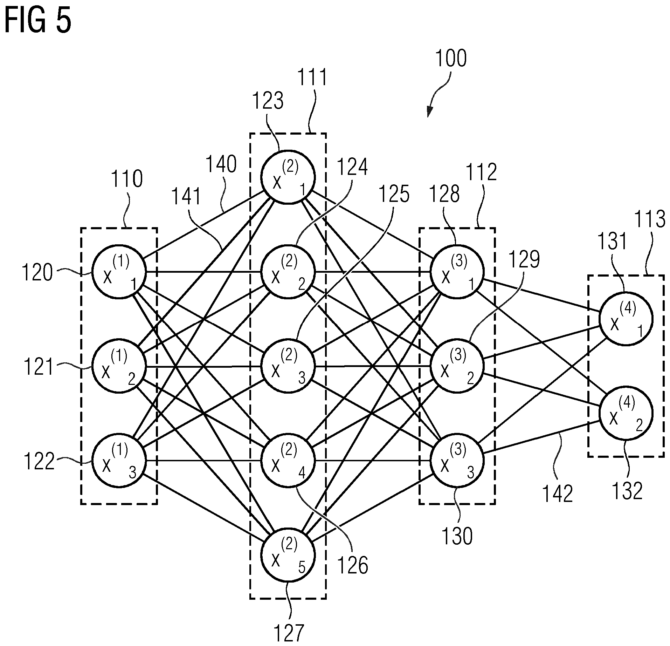

FIG 5 displays an embodiment of an artificial neural network. Alternative terms for "artificial neural network" are "neural network", "artificial neural net" or "neural net".

The trained function may comprise the artificial neural network 100.

The artificial neural network 100 comprises nodes 120, ..., 132 and edges 140, ... , 142, wherein each edge 140, ..., 142 is a directed connection from a first node 120, ... , 132 to a second node 120, ... , 132. In general, the first node 120, ..., 132 and the second node 120, ..., 132 are different nodes 120, ... , 132, it is also possible that the first node 120, ... , 132 and the second node 120, ..., 132 are identical. For example, in Fig. 1 the edge 140 is a directed connection from the node 120 to the node 123, and the edge 142 is a directed connection from the node 130 to the node 132. An edge 140, ... , 142 from a first node 120, ... , 132 to a second node 120, ... , 132 is also denoted as "ingoing edge" for the second node 120, ... , 132 and as "outgoing edge" for the first node 120, ... , 132.

In this embodiment, the nodes 120, ... , 132 of the artificial neural network 100 can be arranged in layers 110, ..., 113, wherein the layers can comprise an intrinsic order introduced by the edges 140, ... , 142 between the nodes 120, ... , 132. In particular, edges 140, ... , 142 can exist only between neighboring layers of nodes. In the displayed embodiment, there is an input layer 110 comprising only nodes 120, ... , 122 without an incoming edge, an output layer 113 comprising only nodes 131, 132 without outgoing edges, and hidden layers 111, 112 in-between the input layer 110 and the output layer 113. In general, the number of hidden layers 111, 112 can be chosen arbitrarily. The number of nodes 120, ... , 122 within the input layer 110 usually relates to the number of input values of the neural network, and the number of nodes 131, 132 within the output layer 113 usually relates to the number of output values of the neural network.

In particular, a (real) number can be assigned as a value to every node 120, ... , 132 of the neural network 100. Here, $x^{(n)}_i$ denotes the value of the i-th node 120, ..., 132 of the n-th layer 110, ... , 113. The values of the nodes 120, ... , 122 of the input layer 110 are equivalent to the input values of the neural network 100, the values of the nodes 131, 132 of the output layer 113 are equivalent to the output value of the neural network 100. Furthermore, each edge 140, ..., 142 can comprise a weight being a real number, in particular, the weight is a real number within the interval [-1, 1] or within the interval [0, 1]. Here, $w^{(m,n)}_{i,j}$ denotes the weight of the edge between the i-th node 120, ... , 132 of the m-th layer 110, ..., 113 and the j-th node 120, ... , 132 of the n-th layer 110, ..., 113. Furthermore, the abbreviation $w^{(n)}_{i,j}$ is defined for the weight $w^{(n,n+1)}_{i,j}$.

In particular, to calculate the output values of the neural network 100, the input values are propagated through the neural network. In particular, the values of the nodes 120, ... , 132 of the (n+1)-th layer 110, ..., 113 can be calculated based on the values of the nodes 120, ... , 132 of the n-th layer 110, ..., 113 by

$$x^{(n+1)}_j = f\left(\sum_i x^{(n)}_i \cdot w^{(n)}_{i,j}\right)$$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes and introducing non-linearities.

In particular, the values are propagated layer-wise through the neural network, wherein values of the input layer 110 are given by the input of the neural network 100, wherein values of the first hidden layer 111 can be calculated based on the values of the input layer 110 of the neural network, wherein values of the second hidden layer 112 can be calculated based in the values of the first hidden layer 111, etc.

In order to set the values $w^{(m,n)}_{i,j}$ for the edges, the neural network 100 has to be trained using training data. In particular, training data comprises training input data and training output data (denoted as $t_i$). For a training step, the neural network 100 is applied to the training input data to generate calculated output data. In particular, the training output data and the calculated output data comprise a number of values, said number being equal with the number of nodes of the output layer.

In particular, a comparison between the calculated output data and the training data is used to recursively adapt the weights within the neural network 100 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ can be recursively calculated as

$$\delta_j^{(n)} = \left(\sum_k \delta_k^{(n+1)} \cdot w_{j,k}^{(n+1)}\right) \cdot f'\left(\sum_i x_i^{(n)} \cdot w_{i,j}^{(n)}\right)$$

based on $\delta^{(n+1)}_j$, if the (n+1)-th layer is not the output layer, and

$$\delta_j^{(n)} = \left(x_k^{(n+1)} - t_j^{(n+1)}\right) \cdot f'\left(\sum_i x_i^{(n)} \cdot w_{i,j}^{(n)}\right)$$

if the (n+1)-th layer is the output layer 113, wherein f' is the first derivative of the activation function, and $y^{(n+1)}_j$ is the comparison training value for the j-th node of the output layer 113.

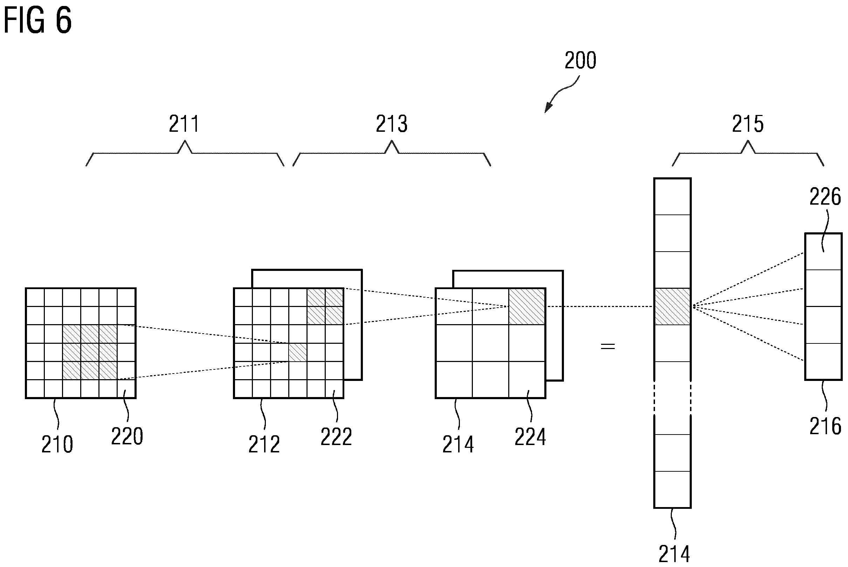

FIG 6 displays an embodiment of a convolutional neural network.

The trained function may comprise the convolutional neural network 200.

In the displayed embodiment, the convolutional neural network comprises 200 an input layer 210, a convolutional layer 211, a pooling layer 212, a fully connected layer 213 and an output layer 214. Alternatively, the convolutional neural network 200 can comprise several convolutional layers 211, several pooling layers 212 and several fully connected layers 213, as well as other types of layers. The order of the layers can be chosen arbitrarily, usually fully connected layers 213 are used as the last layers before the output layer 214.

In particular, within a convolutional neural network 200 the nodes 220, ... , 224 of one layer 210, ..., 214 can be considered to be arranged as a d-dimensional matrix or as a d-dimensional image. In particular, in the two-dimensional case the value of the node 220, ..., 224 indexed with i and j in the n-th layer 210, ..., 214 can be denoted as $x^{(n)}[i,j]$. However, the arrangement of the nodes 220, ... , 224 of one layer 210, ... , 214 does not have an effect on the calculations executed within the convolutional neural network 200 as such, since these are given solely by the structure and the weights of the edges.

In particular, a convolutional layer 211 is characterized by the structure and the weights of the incoming edges forming a convolution operation based on a certain number of kernels. In particular, the structure and the weights of the incoming edges are chosen such that the values $x^{(n)}_k$ of the nodes 221 of the convolutional layer 211 are calculated as a convolution $x^{(n)}_k = K_k * x^{(n-1)}$ based on the values $x^{(n-1)}$ of the nodes 220 of the preceding layer 210, where the convolution * is defined in the two-dimensional case as

$$x_k^{(n)}[i,j] = \left(K_k * x^{(n-1)}\right)[i,j] = \sum_{i'}\sum_{j'} K_k[i',j'] \cdot x^{(n-1)}[i-i', j-j']$$

Here the k-th kernel $K_k$ is a d-dimensional matrix (in this embodiment a two-dimensional matrix), which is usually small compared to the number of nodes 220, ... , 224 (e.g. a 3x3 matrix, or a 5x5 matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespectively of the number of nodes 220, ... , 224 in the respective layer 210, ..., 214. In particular, for a convolutional layer 211 the number of nodes 221 in the convolutional layer is equivalent to the number of nodes 220 in the preceding layer 210 multiplied with the number of kernels.

If the nodes 220 of the preceding layer 210 are arranged as a d-dimensional matrix, using a plurality of kernels can be interpreted as adding a further dimension (denoted as "depth" dimension), so that the nodes 221 of the convolutional layer 221 are arranged as a (d+1)-dimensional matrix. If the nodes 220 of the preceding layer 210 are already arranged as a (d+1)-dimensional matrix comprising a depth dimension, using a plurality of kernels can be interpreted as expanding along the depth dimension, so that the nodes 221 of the convolutional layer 221 are arranged also as a (d+1)-dimensional matrix, wherein the size of the (d+1)-dimensional matrix with respect to the depth dimension is by a factor of the number of kernels larger than in the preceding layer 210.

The advantage of using convolutional layers 211 is that spatially local correlation of the input data can exploited by enforcing a local connectivity pattern between nodes of adjacent layers, in particular by each node being connected to only a small region of the nodes of the preceding layer.

In the displayed embodiment, the input layer 210 comprises 36 nodes 220, arranged as a two-dimensional 6x6 matrix. The convolutional layer 211 comprises 72 nodes 221, arranged as two two-dimensional 6x6 matrices, each of the two matrices being the result of a convolution of the values of the input layer with a kernel. Equivalently, the nodes 221 of the convolutional layer 211 can be interpreted as arranges as a three-dimensional 6x6x2 matrix, wherein the last dimension is the depth dimension.

A pooling layer 212 can be characterized by the structure and the weights of the incoming edges and the activation function of its nodes 222 forming a pooling operation based on a nonlinear pooling function f. For example, in the two dimensional case the values $x^{(n)}$ of the nodes 222 of the pooling layer 212 can be calculated based on the values $x^{(n-1)}$

of the nodes 221 of the preceding layer 211 as

$$x^{(n)}[i,j] = f(x^{(n-1)}[id_1, jd_2], \ldots, x^{(n-1)}[id_1+d_1-1, jd_2+d_2-1])$$

In other words, by using a pooling layer 212 the number of nodes 221, 222 can be reduced, by replacing a number $d_1 \cdot d_2$ of neighboring nodes 221 in the preceding layer 211 with a single node 222 being calculated as a function of the values of said number of neighboring nodes in the pooling layer. In particular, the pooling function f can be the max-function, the average or the L2-Norm. In particular, for a pooling layer 212 the weights of the incoming edges are fixed and are not modified by training.

The advantage of using a pooling layer 212 is that the number of nodes 221, 222 and the number of parameters is reduced. This leads to the amount of computation in the network being reduced and to a control of overfitting.

In the displayed embodiment, the pooling layer 212 is a max-pooling, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

A fully-connected layer 213 can be characterized by the fact that a majority, in particular, all edges between nodes 222 of the previous layer 212 and the nodes 223 of the fully-connected layer 213 are present, and wherein the weight of each of the edges can be adjusted individually.

In this embodiment, the nodes 222 of the preceding layer 212 of the fully-connected layer 213 are displayed both as two-dimensional matrices, and additionally as non-related nodes (indicated as a line of nodes, wherein the number of nodes was reduced for a better presentability). In this embodiment, the number of nodes 223 in the fully connected layer 213 is equal to the number of nodes 222 in the preceding layer 212. Alternatively, the number of nodes 222, 223 can differ.

Furthermore, in this embodiment the values of the nodes 224 of the output layer 214 are determined by applying the Softmax function onto the values of the nodes 223 of the preceding layer 213. By applying the Softmax function, the sum of the values of all nodes 224 of the output layer is 1, and all values of all nodes 224 of the output layer are real numbers between 0 and 1. In particular, if using the convolutional neural network 200 for categorizing input data, the values of the output layer can be interpreted as the probability of the input data falling into one of the different categories.

A convolutional neural network 200 can also comprise a ReLU (acronym for "rectified linear units") layer. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer. Examples for rectifying functions are $f(x) = \max(0,x)$, the tangent hyperbolics function or the sigmoid function.

In particular, convolutional neural networks 200 can be trained based on the backpropagation algorithm. For preventing overfitting, methods of regularization can be used, e.g. dropout of nodes 220, ... , 224, stochastic pooling, use of artificial data, weight decay based on the L1 or the L2 norm, or max norm constraints.

**Claims**

1. A computer-implemented method for determining a respective state (32) of a plurality of predefined anatomical objects (14) of an anatomical region (16), comprising:

    - receiving an image data set (22) of the anatomical region (16) comprising the predefined anatomical objects (14) by an input interface of a data processing device (12),
    - identifying by a computation unit of the data processing device (12) respective image regions (30) of the image data set (22) depicting the predefined anatomical objects (14);
    - applying a function trained by a machine learning algorithm to the respective image regions (30) depicting the respective predefined anatomical objects (14), wherein each of the image regions (30) is examined for a presence of a predetermined pathological finding in the respective anatomical objects (14);
    - determining for each predefined anatomical object (14) of the plurality of predefined anatomical objects (14) a respective state (32) based on the presence of the predetermined pathological finding in the respective predefined anatomical object (14);
    - providing the respective states (32) of the predefined anatomical objects (14) by a second interface of the data processing device (12).

2. The computer-implemented method according to claim 1, comprising:

- generating visual representation data (28) for a display device (24) by the computation unit of the data processing device (12), the visual representation data (28) comprising a visual representation (26) of at least part of the anatomical region (16) visualizing at least one of the image regions (30) depicting the respective anatomical object (14), wherein the visual representation (26) depends on the state (32) of at least one of the predefined anatomical objects (14); and

- providing the visual representation data (28) by the second interface of the data processing device (12).

3. The computer-implemented method of claim 1 or 2, wherein the respective states (32) of the predefined anatomical objects (14) describe a confidence value and/or a probability value of the presence of a predetermined pathological finding in the respective anatomical object (14).

4. The computer-implemented method according to any one of the preceding claims, wherein the anatomical region (16) is a cervical spine and wherein the predefined anatomical objects (14) are vertebral bodies of the cervical spine.

5. The computer-implemented method according to claim 4, wherein the predetermined pathological finding is a fracture of the respective vertebral body of the cervical spine.

6. The computer-implemented method according to any one of the preceding claims, wherein image data set (22) is a set of CT images.

7. The computer-implemented method according to any one of the preceding claims, wherein image data set (22) is a set of MRI images.

8. The computer-implemented method according to any one of the preceding claims, wherein image data set (22) comprises one or more X-ray images.

9. The computer-implemented method according to any one of the preceding claims, comprising:

- acquiring the image data set (22) of the anatomical region (16) using a medical imaging device (18); and
- providing the image data set (22) to the data processing device (12) by the medical imaging device (18).

10. A computer-implemented training method for providing a trained function, comprising:

- receiving input training data comprising image data sets (22) of respective anatomical regions (16) comprising respective predefined anatomical objects (14), wherein the image data sets (22) comprise image regions (30) depicting the predefined anatomical objects (14), by a first training interface of a training device;
- receiving output training data by a second training interface of the training device, wherein the output training data comprise for each of the image regions (30) an indication of a presence of a predetermined pathological finding in the respective anatomical objects (14);
- training a function based on the input training data and the output training data by a training computation unit of the training device; and
- providing the trained function by a third training interface of the training device.

11. A data processing device (12), comprising

- a first interface, configured for receiving an image data set (22) of the anatomical region (16) comprising predefined anatomical objects (14),
- a second interface, configured for providing respective states (32) of the predefined anatomical objects (14),
- a computation unit, configured for identifying respective image regions (30) of the image data set (22) depicting the predefined anatomical objects (14), applying a function trained by a machine learning algorithm to the respective image regions (30) depicting the respective predefined anatomical objects (14), wherein each of the image regions (30) is examined for a presence of a predetermined pathological finding in the respective anatomical objects (14), and determining for each of the predefined anatomical objects (14) a respective state (32) based on the presence of the predetermined pathological finding in the respective predefined anatomical object (14).

12. An imaging system (10) comprising an imaging device and a data processing device (12) according to claim 11

13. A computer program comprising instructions which, when the program is executed by a computer, cause the computer to carry out the method of one of the claims 1 to 9 or the method of claim 10.

14. A computer-readable medium comprising instructions which, when executed by a computer, cause the computer to carry out the method of one of the claims 1 to 9 or the method of claim 10.

FIG 1

FIG 2

| 32 | |
|----|-----|
| C1 | – 12% |
| C2 | – 5% |
| C3 | – 75% |
| C4 | – 7% |
| C5 | – 97% |
| C6 | – 8% |
| C7 | – 10% |

# FIG 3

A1
A2
A3
A4
A5

# FIG 4

B1
B2
B3
B4

FIG 5

FIG 6

<table>
<tr><td>Europäisches Patentamt<br>European Patent Office<br>Office européen des brevets</td><td align="center">**EUROPEAN SEARCH REPORT**</td><td>**Application Number**<br><br>**EP 24 46 5581**</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Yilmaz Eren Bora ET AL: "Automated Deep Learning-Based Detection of Osteoporotic Fractures in CT Images : 12th International Workshop, MLMI 2021, Held in Conjunction with MICCAI 2021, Strasbourg, France, September 27, 2021, Proceedings" In: "Machine Learning in Medical Imaging : 12th International Workshop, MLMI 2021, Held in Conjunction with MICCAI 2021, Strasbourg, France, September 27, 2021, Proceedings", 1 January 2021 (2021-01-01), Springer International Publishing, XP093257484, ISSN: 0302-9743 ISBN: 978-3-030-87589-3 vol. 12966, pages 376-385, DOI: 10.1007/978-3-030-87589-3_39, Retrieved from the Internet: URL:https://link.springer.com/content/pdf/10.1007/978-3-030-87589-3_39> * abstract * * pages 3,4,6; figures 1,2 * | 1-14 | INV.<br>G06T7/00<br>A61B6/50<br>G06V10/82 |
| | ----- | | |
| A | LI SHEN ET AL: "Using Artificial Intelligence to Diagnose Osteoporotic Vertebral Fractures on Plain Radiographs", JOURNAL OF BONE AND MINERAL RESEARCH, BLACKWELL SCIENCE, INC, US, vol. 38, no. 9, 2 August 2023 (2023-08-02), pages 1278-1287, XP072502299, ISSN: 0884-0431, DOI: 10.1002/JBMR.4879 * abstract * * page 3 - page 5; figures 2,3 * | 1-14 | TECHNICAL FIELDS SEARCHED (IPC)<br><br>G06T<br>A61B<br>G06V |
| | ----- | | |
| A | CN 114 494 192 A (SOUTHWEST TRAFFIC UNIV) 13 May 2022 (2022-05-13) * abstract * * paragraphs [0023], [0034], [0036] * * paragraphs [0092], [0097] * | 1-14 | |
| | ----- | | |

The present search report has been drawn up for all claims

| Place of search | Date of completion of the search | Examiner |
|---|---|---|
| Munich | 10 March 2025 | Katsoulas, Dimitrios |

EPO FORM 1503 03.82 (P4C01)

## ANNEX TO THE EUROPEAN SEARCH REPORT
## ON EUROPEAN PATENT APPLICATION NO.

EP 24 46 5581

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2025

| Patent document cited in search report | Publication date | Patent family member(s) | Publication date |
|---|---|---|---|
| CN 114494192 A | 13-05-2022 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20220309667 A1
- US 11275976 B2
- US 20220293247 A1